# EUROPÄISCHE PATENTANMELDUNG

(11) **EP 4 529 227 A1**
(43) Veröffentlichungstag der Anmeldung: **26.03.2025**
(21) Anmeldenummer: 24195277.9
(22) Anmeldetag: 20.08.2024
(51) Int. Cl.: H04R 25/00, A61M 21/00, A61M 21/02, H04R 1/10, H04R 5/033, H04R 5/04, A61B 5/375, A61B 5/38, A61B 5/00, H04S 1/00, H04S 7/00

(54) **HÖRSYSTEM SOWIE VERFAHREN ZUR ERZEUGUNG VON MONAURALEN ODER BINAURALEN BEATS**

(30) Priorität: 19.09.2023 DE 102023209094
(71) Anmelder: Sivantos Pte. Ltd., Singapore 539775 (SG)
(72) Erfinder: FRÖHLICH, Matthias, 91058 Erlangen (DE); HAIN, Jens, 91058 Erlangen (DE); HÖGL, Florian, 91058 Erlangen (DE); NAUMANN, Frank, 91058 Erlangen (DE)
(74) Vertreter: FDST Patentanwälte

(57) **Zusammenfassung**

Bei dem Hörsystem (2) handelt es sich insbesondere um ein binaurales Hörsystem (2), welches zur Erzeugung von monauralen oder binauralen Beats ausgebildet ist und hierzu zumindest ein Hörgerät (4A, 4B) mit einem Haupt-Signalpfad (8) aufweist. Das Hörgerät (4A, 4B) weist weiter einen Neben-Signalpfad (26) auf, mit einem Signalgenerator (28) zur Erzeugung eines ersten Beatsignals (B1), wobei das erste Beatsignal (B1) zu einem weiteren Signal (B2) um eine Beatfrequenz frequenzverschoben ist, derart, dass im Betrieb beim Nutzer der Effekt des monauralen oder binauralen Beats erzeugt wird. Der Signalgenerator (28) ist derart eingerichtet, dass das erste Beatsignal (B1) zumindest in bestimmten Betriebsmodi des Hörgerätes (4A, 4B) zumindest eine als Umgebungssignal (Su) bezeichnete Signalkomponente aufweist, die auf Basis des elektrischen Eingangssignal (E1) und damit auf Basis der Umgebungsgeräusche erzeugt ist. Bevorzugt setzt sich das Beatsignal (B1, B2) aus unterschiedlichen Signalkomponenten zusammen, deren Gewichtung (G) bevorzugt in Abhängigkeit eines Signalpegels des Eingangssignals eingestellt wird.

## Beschreibung

Die Erfindung betrifft ein insbesondere binaurales Hörsystem sowie ein Verfahren zur Erzeugung von monauralen oder binauralen Beats mithilfe eines derartigen Hörsystems.

Unter Hörsystem wird vorliegend ein System mit zumindest einem Hörgerät und bei einem binauralen Hörsystemen ein System mit zwei Hörgeräten verstanden. Bei den Hörgeräten handelt es sich um elektronische Geräte, die ein Nutzer auf dem Ohr, hinter dem Ohr oder im Ohr trägt. Bei dem Hörsystem handelt es sich insbesondere um ein Hörhilfesystem, welches dazu eingerichtet ist, einen nutzerspezifischen Hörschaden einer hörgeschädigten Person zu kompensieren.

Ein derartiges Hörgerät weist jeweils üblicherweise einen Eingangswandler auf, welcher ein akustisches oder auch ein gestreamtes Eingangssignal zunächst in ein elektrisches Eingangssignal wandelt, welches nachfolgend mittels einer insbesondere digitalen Signalverarbeitungseinheit zu einem elektrischen Ausgangssignal aufbereitet wird, welches einem Ausgangswandler zugeführt wird, welcher das elektrische Ausgangssignal in ein akustisches Ausgangssignal wandelt, welches an das Ohr des Hörers abgegeben wird.

Unter monauralen Beats oder binauralen Beats werden bekannte Effekte einer Sinneswahrnehmung im menschlichen Gehirn verstanden, bei der der Nutzer einen pulsierenden Ton (Beat) wahrnimmt, wenn ihm zwei gleiche akustische Signale mit leicht voneinander abweichender Frequenz dargeboten werden. Bei binauralen Beats werden dem Nutzer auf zwei Kanälen, nämlich auf dem linken Ohr und auf dem rechten Ohr, die beiden zueinander frequenzverschobenen akustischen Signale dargeboten. Bei monauralen Beats werden die beiden frequenzverschobenen akustischen Signale lediglich auf einem Kanal dargeboten.

Mit derartigen monauralen oder binauralen Beats lassen sich bestimmte Trainingseffekte für das Gehirn erreichen.

Ausgehend hiervon liegt der Erfindung die Aufgabe zugrunde, mit Hilfe eines Hörsystems akustische Ausgangssignal zu erzeugen, die geeignet sind, beim Benutzer eine angenehme Wahrnehmung von monauralen oder binauralen Beats zu erzeugen.

Die Aufgabe wird gemäß der Erfindung gelöst durch ein Hörsystem, insbesondere ein binaurales Hörsystem mit den Merkmalen des Anspruchs 1 sowie durch ein Verfahren zur Erzeugung von monauralen oder binauralen Beats mithilfe eines solchen Hörsystems. Im Hinblick auf das Hörsystem angeführte Vorteile und bevorzugten Ausgestaltungen sind sinngemäß auch auf das Verfahren und umgekehrt zu übertragen.

Bei dem Hörsystem handelt es sich insbesondere um ein Hörhilfesystem, welches nutzerspezifisch eingerichtet ist und zur Kompensation eines nutzerspezifischen Hörschadens ausgebildet ist.

Das Hörsystem weist zumindest ein Hörgerät und bevorzugt, speziell bei der Ausgestaltung als binaurales Hörsystems, zwei Hörgeräte auf, wobei
- das zumindest eine Hörgerät einen Haupt-Signalpfad aufweist, mit einem Eingangswandler zur Erzeugung eines elektrischen Eingangssignals aus einem beispielsweise akustischen oder gestreamten Eingangssignal, mit einer insbesondere digitalen Signalverarbeitungseinheit zur Verarbeitung des elektrischen Eingangssignals und zur Erzeugung eines elektrischen Ausgangssignals, sowie mit einem Ausgangswandler, welcher basierend auf dem elektrischen Ausgangssignal ein akustisches Ausgangssignal erzeugt,
- das zumindest eine Hörgerät weiterhin einen Neben-Signalpfad aufweist, mit jeweils einem Signalgenerator zur Erzeugung eines ersten Beatsignals, wobei das erste Beatsignal zu einem weiteren Signal um eine Beatfrequenz frequenzverschoben ist, derart, dass im Betrieb beim Nutzer der Effekt des monauralen oder binauralen Beats erzeugt wird,
- das zumindest eine Hörgerät einen Summierer aufweist, welcher zumindest das erste Beatsignal auf ein Signal des Haupt-Signalpfades aufsummiert, so dass das akustische Ausgangssignal gebildet ist durch einen normalen Audiosignalanteil und einen Beatsignalanteil,
- der Signalgenerator (28) mit dem ersten Signalpfad (8) verbunden ist und derart eingerichtet ist, dass das erste Beatsignal (B1) zumindest in bestimmten Betriebsmodi des Hörgerätes (4A, 4B) zumindest eine als Umgebungssignal bezeichnete Signalkomponente aufweist, die auf Basis des elektrischen Eingangssignal (E1) erzeugt ist.

Im Falle eines binauralen Hörsystems weisen die beiden Hörgeräte jeweils eine Kommunikationseinheit auf, die eine Kommunikationsschnittstelle bildet, über die die beiden Hörgeräte in bekannter Weise wechselweise miteinander kommunizieren und Daten austauschen. Insbesondere ist hierzu eine Bluetooth-Verbindung ausgebildet.

Bei einem Hörsystem mit zwei Hörgeräten, insbesondere bei einem binauralen Hörsystem, weist das zweite Hörgerät ebenfalls einen Haupt-Signalpfad mit den zuvor aufgeführten Komponenten auf.

In bevorzugter Ausgestaltung, jedoch nicht zwingend, weist ein derartiges zweites Hörgerät auch einen Neben-Signalpfad auf, in dem ebenfalls ein Signalgenerator zur Erzeugung eines zweiten Beatsignals angeordnet ist. In dieser Ausführungsvariante sind die beiden Beatsignale um die Beatfrequenz zueinander frequenzverschoben. Auch das zweite Hörgerät weist in diesem Fall einen Summierer auf, welcher das zweite Beatsignal auf ein Signal des ersten Signalpfades aufsummiert. Dadurch wird auch im zweiten Hörgerät ein akustisches Ausgangssignal erzeugt, welches gebildet ist durch einen normalen Audiosignalanteil und einen Beatsignalanteil auf Basis des zweiten Beatsignals.

Unter normalen Audiosignalanteil wird im akustischen Ausgangssignal derjenige Signalanteil verstanden, welcher sich ohne die Aufsummierung des Beatsignals ergeben würde.

Unter Beatfrequenz wird vorliegend die Frequenz verstanden, um die die beiden Beatsignale zueinander frequenzverschoben sind. Die Beatfrequenz liegt dabei typischerweise im Bereich von 0,5 Hz bis 60 Hz, vorzugsweise maximal bis 55 Hz und häufig im Bereich zwischen 3 Hz und 30 Hz.

Zur Erzeugung des frequenzverschobenen Beatsignals weist zumindest der eine Signalgenerator einen Frequenzverschieber auf, welcher also ein durch den Signalgenerator erzeugtes oder bereitgestelltes Signal (z.B. Umgebungssignal Sinussignal, schmalbandiges Rauschen, Signalanteil) um die Beatfrequenz verschiebt und damit das frequenzverschobene Beatsignal erzeugt.

Durch die Verwendung des Umgebungssignals, also des von dem Eingangswandler erfassten akustischen Eingangssignals als Basis für die Erzeugung des (frequenzverschobenen) ersten Beatsignals, wird der Vorteil erzielt, dass die Generierung der Beatsignale und damit für die Erzeugung der Wahrnehmung der Beats die Umgebungsgeräusche als Basis herangezogen werden. Dies wird von dem Nutzer als besonders angenehm empfunden, da in diesem Fall keine zusätzlichen, fremden Signalkomponenten enthalten sind. Gleichzeitig wird jedoch weiterhin die gewünschte Wahrnehmung der monauralen oder binauralen Beats erreicht.

Der Signalverlauf des Umgebungssignals entspricht dabei dem Signalverlauf zumindest von einem ausgewählten Frequenzbandes des Eingangssignals.

Die Ausgestaltung eines Neben-Signalpfades beim zweiten Hörgerät ist nicht zwingend erforderlich, auch nicht bei binauralen Hörgeräten und / oder bei der Erzeugung von binauralen Beats.

Im Fall der Erzeugung von monauralen Beats wird beispielsweise lediglich innerhalb des zumindest einen Hörgerätes ein normaler Signalanteil auf Basis des Eingangssignals und ein frequenzverschobener Signalanteil auf Basis des Eingangssignals als Beatsignale bereitgestellt, die dann zur Wahrnehmung der monauralen Beats führen. Auch bei der Erzeugung von binauralen Beats ist es in einer Ausführungsvariante ausreichend, wenn beispielsweise im Signalgenerator des ersten Hörgeräts das erste Beatsignal auf Basis des Eingangssignals lediglich durch eine Frequenzverschiebung des Eingangssignals oder von Signalanteilen des Eingangssignals erzeugt und als das Umgebungssignal bereitgestellt wird. Der binaurale Effekt wird beispielsweise durch das Audiosignal (akustisches Ausgangssignal) am zweiten Hörgerät und dem durch den frequenzverschobenen Beatsignalanteil (auf Basis des ersten Beatsignals) im akustischen Ausgangssignal des ersten Hörgeräts erzeugt.

Insbesondere bei solchen Varianten wird im zweiten Hörgerät auf einen Neben-Signalpfad verzichtet und / oder es wird lediglich im ersten Hörgerät ein frequenzverschobenes Beatsignal erzeugt.

Bevorzugt ist jedoch die Variante, bei dem beide Hörgeräte einen Signal-Nebenpfad und jeweils einen Signalgenerator aufweisen. In diesem Fall weist daher in beiden Hörgeräten die von dem jeweiligen Signalgenerator erzeugten Beatsignale zumindest das Umgebungssignal als Signalkomponente auf. In einer Variante bestehen die Beatsignale aus dem Umgebungssignal. Die Beatsignale und damit auch die Umgebungssignale der beiden Beatsignale sind dabei identisch, bis auf die Frequenzverschiebung um die Beatfrequenz. In einigen Varianten können sich die beiden Beatsignale / Umgebungssignale auch im Hinblick auf Ihre Amplitude unterscheiden.

Sofern vorliegend von einem Beatsignal gesprochen wird, so wird hierunter allgemein ein Signal verstanden, welches im akustischen Ausgangssignal zu einem Signalanteil beiträgt, welcher zur Erzeugung des gewünschten Effekts des monauralen oder binauralen Beats beiträgt. Zumindest in einigen bestimmten Betriebsmodi des Hörgerätes basieren diese Signalanteile (Umgebungssignal) auf dem Umgebungsgeräuschen, also auf den akustischen Eingangssignalen.

Gemäß einer bevorzugten Ausgestaltung weist das zumindest eine Hörgerät eine Analyseeinheit zur Analyse des Eingangssignals auf und das Beatsignal setzt sich aus ein oder mehreren Signalkomponenten zusammen. Der Signalgenerator ist hierbei dazu eingerichtet, die Zusammensetzung des Beatsignals auf Basis der Analyse des Eingangssignals vorzunehmen.

Die verschiedenen Signalkomponenten, aus denen das Beatsignal in Abhängigkeit der Analyse des Eingangssignals und damit der Umgebungsgeräusche zusammengesetzt ist, sind dabei bevorzugt ausgewählt aus dem Umgebungssignal, einem Sinussignal und einem insbesondere schmalbandigen Rauschsignal. Ein jedes dieser Signale bildet daher einen möglichen Signalanteil des jeweiligen Beatsignals.

Unter schmalbandigem Rauschsignal wird dabei ein Rauschen verstanden, welches sich über ein Frequenzband erstreckt, welches vorzugsweise lediglich eine Bandbreite von 100 Hz bis 300 HZ aufweist. Bevorzugt liegt das Rauschsignal zwischen 250 Hz bis 5 kHz.

Diese weiteren Signale neben dem Umgebungssignal können auch allgemein als Trägersignale bezeichnet werden, die unabhängig von dem Eingangssignal sind.

Bevorzugt weist der jeweilige Signalgenerator für jede Signalkomponente ein individuelles, für die Signalkomponente konfiguriertes Generatorteil auf.

Die Auswahl der Signalanteile und die Zusammensetzung des Beatsignals erfolgt vorzugsweise in Abhängigkeit eines Signalpegels des (akustischen) Eingangssignals. Das jeweilige Beatsignal kann dabei aus jeweils nur einer Signalkomponente, aus mehreren Signalkomponenten oder auch aus allen Signalkomponenten bestehen.

Diese Ausgestaltung beruht dabei auf der Erkenntnis, dass für unterschiedliche Signalpegel des Eingangssignals unterschiedliche Arten von Beatsignalen für den Nutzer besonders angenehm sind.

So hat sich gezeigt, dass für geringe Signalpegel ein einzelner Ton als Beatsignals basierend auf einem Sinussignal als störender oder unangenehmer empfunden wird als ein insbesondere schmalbandiges Rauschen. Mit zunehmendem Signalpegel kehrt sich diese Situation um und das Sinussignal wird als angenehmer empfunden. Zu höheren Signalpegeln schließlich wird die Verwendung von Umgebungssignalen als besonders angenehm empfunden, da andernfalls beispielsweise sehr laute Sinussignale als zusätzliche Signale erzeugt werden müssten, die dann als störend empfunden werden.

Bei dem Signalpegel des Eingangssignals handelt es sich allgemein um einen Kennwert für den Signalpegel, also für die Amplitude des akustischen oder auch des gestreamten Eingangssignals, welches vom Eingangswandler erfasst wird. Die Bestimmung des (momentanen) Signalpegels erfolgt dabei regelmäßig auf Basis einer Auswertung des gewandelten, elektrischen Eingangssignals. Speziell handelt es sich bei der Bestimmung des Signalpegels um eine Schätzung. Mit der Bestimmung des Signalpegels wird daher insgesamt ein Signalpegel und damit eine Amplitude der momentanen Umgebungsgeräusche bestimmt bzw. abgeschätzt.

Zur Bestimmung des Signalpegels können unterschiedliche Verfahren eingesetzt werden. Beispielsweise wird ein breitbandiger Signalanteil des Eingangssignals, beispielsweise die komplette Bandbreite des hörbaren Frequenzspektrums oder nur ein Teilband ausgewertet. Hierbei werden beispielsweise die Signalpegel in verschiedenen Frequenzbändern gemittelt und der gemittelte Signalpegel wird als der Signalpegel des Eingangssignals betrachtet. Alternativ wird das Frequenzband mit dem höchstem Signalpegel herangezogen und dieser maximale Signalpegel wird als der Signalpegel des Eingangssignals betrachtet.

Auch besteht die Möglichkeit, eine Referenzfrequenz festzulegen, bei der der Signalpegel als Referenzpegel und damit als Signalpegel des Eingangssignals bestimmt wird. Bei der Referenzfrequenz handelt es sich beispielsweise um eine mittlere Frequenz eines ausgewählten Frequenzbandes oder um die Frequenz mit dem höchsten Signalpegel. Das Frequenzband und / oder die Referenzfrequenz werden beispielsweise fest vorgegeben oder alternativ dynamisch jeweils anhand des momentanen maximalen Signalpegels bestimmt.

In zweckdienlicher Weiterbildung ist weiterhin vorgesehen, dass in Abhängigkeit des ermittelten Signalpegels eine unterschiedliche Gewichtung der Signalkomponenten vorgenommen wird. Dies bedeutet, dass sich die gewichtete Zusammensetzung des jeweiligen Beatsignals mit dem Signalpegel verändert.

Unter Gewichtung wird vorzugsweise der Signalpegel der jeweiligen Signalkomponente im akustischen Ausgangssignal verstanden. Erstreckt sich die jeweilige Signalkomponente über ein Frequenzband, wie beispielsweise bei dem Rauschen oder dem Umgebungssignal, so wird die Gewichtung durch den maximalen Signalpegel innerhalb des Frequenzbandes bestimmt oder alternativ durch einen mittleren Signalpegel innerhalb des Frequenzbandes.

Die Gewichtung und damit der Signalpegel der verschiedenen Signalkomponenten wird insbesondere mithilfe von zumindest einem einstellbaren Verstärkerelement eingestellt.

Bevorzugt weist das Verstärkerelement dabei für jede der Signalkomponenten eine Verstärker-Teilkomponente auf, um die individuellen Gewichtungsfaktoren und damit Verstärkungsfaktoren unabhängig voneinander einstellen zu können. Zweckdienlicherweise ist einem jeden individuellen Generatorteil, welcher für eine jeweilige Signalkomponente eingerichtet ist, eine jeweilige Verstärker-Teilkomponente zugeordnet.

Auf Basis der zuvor beschriebenen Erkenntnis im Zusammenhang mit der Wirkung der verschiedenen Signalkomponenten in Abhängigkeit speziell des Signalpegels des Eingangssignals ist insbesondere vorgesehen, dass die Gewichtung des Umgebungssignale mit zunehmendem Signalpegel zunimmt.

Alternativ oder ergänzend ist bei einem geringen Signalpegel die Gewichtung des Umgebungssignals geringer als die der weiteren Signalkomponenten, und zwar insbesondere als jede einzelne der anderen Signalkomponenten. Mit zunehmendem Signalpegel nimmt die Gewichtung des Umgebungssignals zu und übersteigt schließlich beispielsweise in einem mittleren Pegelbereich jeweils die anderen Signalkom ponenten.

In bevorzugter Ausbildung wird bei einem unteren, leisen Pegelbereich des Signalpegels des Eingangssignals das Rauschsignal, in einem mittleren Pegelbereich das Sinussignal und bei einem hohen Pegelbereich das Umgebungssignal am höchsten gewichtet. Insgesamt ergibt sich daher ein qualitativer Gewichtungs-Verlauf, bei dem bei einem leisen Signalpegel das insbesondere schmalbandige Rauschen dominiert, nachfolgend bei einem mittlerem Pegelbereich das Sinussignal und schließlich bei einem hohen Pegelbereich das Umgebungssignal dominiert. Unter einem leisen Signalpegel wird insbesondere ein Signalpegel bis 50 dBSPL oder auch bis 55 dBSPL (decibel Sound Pressure Level - dB-Schalldruckpegel) verstanden. Unter einem mittlerem Signalpegel wird ein Pegelbereich zwischen 50 / 55 dBSPL - 75 dBSPL oder auch bis 80 dBSPL verstanden und unter lautem Signalpegel wird insbesondere ein Pegel oberhalb von 75 dBSPL oder auch von 80 dBSPL verstanden

In bevorzugter Ausgestaltung wird die Analyse wiederkehrend und insbesondere kontinuierlich und fortlaufend durchgeführt wird. Ein Zeitintervall zwischen zwei aufeinanderfolgenden Analyseschritten ist dabei vorzugsweise kleiner 300 ms und liegt beispielsweise bei 100 ms. Das Zeitintervall liegt allgemein vorzugsweise zwischen 100 ms bis 300 ms. Auf Basis jeder dieser kontinuierlich und wiederholt durchgeführten Analysen wird dann die Zusammensetzung des Beatsignals in Abhängigkeit der momentanen Analyse angepasst, d. h. die Zusammensetzung des Beatsignals und damit die Gewichtung der verschiedenen Signalkomponente verändert sich im Laufe der Zeit. Die Zusammensetzung wird daher quasi dem Pegelverlauf des Eingangspegels kontinuierlich nachgeführt.

Neben der Analyse und Bestimmung des Signalpegels des Eingangssignals werden bevorzugt weitere Parameter herangezogen, anhand derer sich die Zusammensetzung des Beatsignals bestimmt. Solche Parameter sind beispielsweise momentane Zustandswerte des jeweiligen Nutzers, beispielsweise der Herzschlag, Bewegungen oder Beschleunigungen von Körperteilen usw. Diese Parameter werden wahlweise mit einem Zusatzgerät oder auch mittelbar aus den Eingangssignalen abgeleitet. Diese Ausgestaltung beruht auf der Überlegung, dass auch in bestimmten Zustands-Situationen des Nutzers, beispielsweise beim Schlafen, beim Sport treiben usw. verschiedene Signalkomponente unterschiedlich gut für den angestrebten Effekt geeignet sind.

In zweckdienlicher Ausgestaltung handelt es sich bei dem Umgebungssignal um ein bandgefiltertes Umgebungssignal. Das Hörgerät weist entsprechend auch einen geeigneten Bandfilter auf, sodass das Umgebungssignal über diesen aus dem erfassten Eingangssignal herausgefiltert wird und eine vorgegebene Bandbreite aufweist. Dies beruht auf der Überlegung, dass für die Erzeugung der Wahrnehmung der Beats nur ein bestimmte Frequenzbereich besonders geeignet ist.

Bevorzugt weist das bandgefilterte Umgebungssignal eine Bandbreite im Bereich von 100 Hz bis 400 Hz auf. Weiterhin liegt das Umgebungssignal vorzugsweise in einem mittleren Bandbereich zwischen 200 Hz und 1000 Hz, und weist dabei insbesondere die zuvor genannte Bandbreite auf.

Gemäß einer bevorzugten Weiterbildung werden Parameter des bandgefilterten Umgebungssignals, wie Bandbreite, mittlere Frequenz oder untere bzw. obere Bandfrequenz, in Abhängigkeit eines benutzerspezifischen Audiograms, also in Abhängigkeit eines benutzerspezifischen Hörschadens eingestellt. Speziell wird die Bandbreite und/oder die mittlere Frequenz derart gewählt, dass sie außerhalb eines Frequenzbandes ist, bei dem der Nutzer - im Vergleich zu anderen Frequenzbändern - große Hördefizite hat, wo eine hohe Verstärkung erforderlich ist.

Das Frequenzband des Umgebungssignals wird daher insbesondere in einen solchen Frequenzbereich gelegt, wo eine vergleichsweise geringe nutzerspezifische Verstärkung erforderlich ist.

In zweckdienlicher Ausgestaltung ist das Hörsystems dahingehend eingerichtet, dass sich die Werte der Parameter des bandgefilterten Umgebungssignals, wie Bandbreite, mittlere Frequenz oder untere bzw. obere Bandfrequenz im Zeitverlauf und insbesondere in Abhängigkeit des momentanen Eingangssignals variieren. Die Parameterwerte werden dabei beispielsweise in Abhängigkeit des Signalpegels gewählt. Speziell wird beispielsweise eine mittlere Frequenz des Umgebungssignals derart gewählt, dass diese außerhalb einer Frequenz oder eines Frequenzbandes mit dem höchsten Signalpegel des Eingangssignals liegt. Dies dient dazu, um Maskierungseffekte zu vermeiden.

Insgesamt handelt sich daher bei dem bandgefilterten Umgebungssignal um ein im Zeitverlauf dynamisches Umgebungssignal, dessen Parameterwerte variieren und zwar insbesondere in Abhängigkeit von dem Eingangssignal, speziell in Abhängigkeit des Signalpegels des Eingangssignals.

In bevorzugter Weiterbildung ist vorzugsweise in jedem Hörgerät eine Einstellvorrichtung dem jeweiligen Signalgenerator zugeordnet, wobei die Einstellvorrichtung das zuvor beschriebene, einstellbare Verstärkerelement aufweist. Auch die zuvor erwähnte Analyseeinheit ist Teil dieser Einstellvorrichtung. Weiterhin ist die Einstellvorrichtung dazu eingerichtet, einen Signalpegel zumindest des ersten Beatsignals und vorzugsweise von beiden Beatsignalen in Abhängigkeit eines momentanen, aktuellen Signalpegels des Eingangssignals einzustellen.

Durch diese Maßnahme wird der Signalpegel des Beatsignals dem momentanen Signalpegel des Eingangssignals insbesondere kontinuierlich nachgeführt. Die Durchführung der Analyse und die entsprechende Einstellung des Signalpegels der Beatsignale erfolgen dabei vorzugsweise in Zeitperioden kleiner 500 ms und beispielsweise alle 100 ms.

Die Nachführung des Signalpegels des Beatsignals führt zu einer besonders angenehmen Wahrnehmung der monauralen oder binauralen Beats.

In bevorzugter Ausgestaltung weist das Hörsystem ein nutzerspezifisches Einstellelement auf, über welches eine manuelle Einstellung des Beatsignals, insbesondere des Signalpegels des Beatsignals möglich ist. Das Hörsystem weist hierzu beispielsweise eine Bedien-Schnittstelle auf, insbesondere in Form eines tragbaren Gerätes wie beispielsweise ein Smartphone, auf dem eine Anwendung installiert ist, über die Einstellungen für das jeweilige Hörgerät vorgenommen werden können. Dieses tragbare Gerät ist dabei Teil des Hörsystems. Alternativ oder ergänzend können auch unmittelbar an zumindest einem Hörgerät Bedienelemente angeordnet sein, über die die Einstellung erfolgen kann.

Über diese manuellen Einstellmöglichkeiten bietet sich daher für den Nutzer die Möglichkeit, Parameter, welche die Beats beeinflussen selber zumindest nach zu justieren, um ein möglichst angenehmes Hörerempfinden zu erhalten. Die Parameter sind dabei insbesondere die Beatfrequenz und / oder der Verstärkungsfaktor für das Verstärkerelement bzw. der Signalpegel des jeweiligen Beatsignals, insbesondere der Pegelabstand im Vergleich zum normalen Audiosignalanteil im akustischen Ausgangssignal.

In bevorzugter Weiterbildung weist das Hörsystem einen Statuserkenner auf, ist also zur automatischen Erkennung eines momentanen Status des Nutzers ausgebildet. Weiterhin ist das Hörsystem, insbesondere die Einstellvorrichtung dazu eingerichtet, in Abhängigkeit des identifizierten momentanen Status das zumindest eine Beatsignal einzustellen. Unter Status des Nutzers wird insbesondere ein momentaner körperlicher Zustand des Nutzers verstanden, welcher beispielsweise durch eine Bestimmung des momentanen Pulses des Nutzers und/oder durch Beschleunigungssensoren ermittelt wird. Die Bestimmung des momentanen Status kann dabei entweder mit Hilfsgeräten oder direkt auch mithilfe des Hörgeräts vorgenommen werden. Beispielsweise können im Hörgerät Beschleunigungssensoren integriert sein oder das Hörgerät ist dazu ausgelegt, aus akustischen Signalen, z.B. Körperschall-Signale, den momentanen Puls des Hörgeräteträgers zu bestimmen.

Insbesondere wird ein Schlafmodus identifiziert, insbesondere im Vergleich zu einem Wach-Modus, wobei dann für die unterschiedlichen Modi die Parameter unterschiedlich eingestellt werden. Unter Parameter für das Beatsignal werden wiederum insbesondere die Beatfrequenz (also die Größe der Frequenzverschiebung zwischen den Beatsignalen) und / oder der Signalpegel verstanden. So wird beispielsweise bei einem identifizierten Schlafmodus der Signalpegel im Vergleich zu einem Wach-Modus reduziert. Ergänzend oder alternativ werden für den Wachmodus und dem Schlafmodus unterschiedliche Beatfrequenzen eingestellt. Es ist nämlich bekannt, dass unterschiedliche Beatfrequenzen unterschiedliche Auswirkungen und Trainingseffekte für das Gehirn aufweisen.

In bevorzugter Ausgestaltung wird - beispielsweise im Schlafmodus - die Beatfrequenz auf einen Bereich zwischen 35 Hz bis 45 Hz und insbesondere auf 40 Hz eingestellt. Untersuchungen haben gezeigt, dass ein derartige Beatfrequenzen speziell im Schlafmodus besondere Vorteile aufweisen.

Ausführungsbeispiele der Erfindung werden nachfolgend anhand der Figuren näher erläutert, dabei zeigen
- FIG 1: eine vereinfachten Blockbild-Darstellungen eine Ausführungsvariante eines binauralen Hörsystems zur Erläuterung der Nachführung eines Signalpegels von Beatsignalen in Abhängigkeit des Signalpegels eines Eingangssignal,
- FIG 2: eine ausschnittsweise Darstellung basierend auf dem Hörsystem gemäß FIG 1 zur Erläuterung der Zusammensetzung der Beatsignale aus verschiedenen Signalkomponenten, sowie
- FIG 3: eine Darstellung eines qualitativen Verlaufs der Gewichtung der verschiedenen Signalkomponenten in Abhängigkeit des Signalpegels des Eingangssignals.

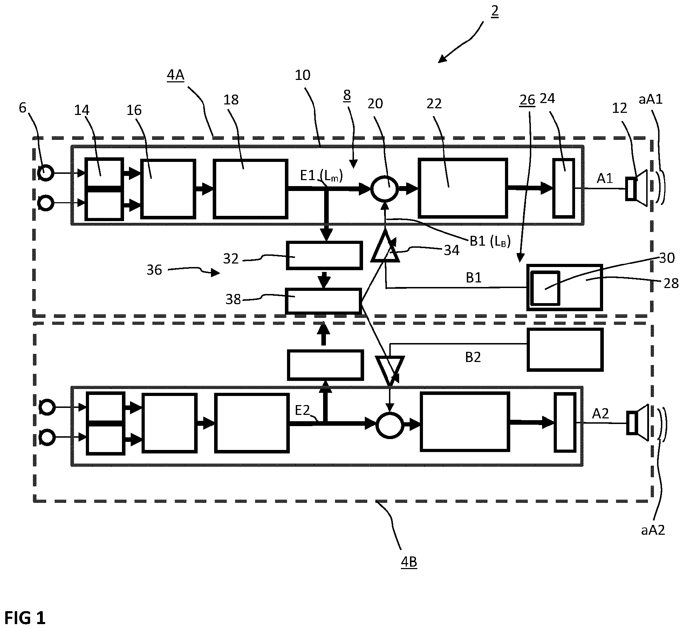

Ein in FIG 1 dargestellte Hörsystem 2 ist als ein Hörhilfesystem ausgebildet, welches zur Kompensation eines benutzerspezifischen Hörschadens eingerichtet ist. Es handelt es sich hierbei um ein binaurales Hörsystem 2 mit zwei Hörgeräten 4A, 4B welche jeweils über eine hier nicht näher dargestellte drahtlose Kommunikationseinheit, insbesondere eine Bluetooth-Kommunikations-Schnittstelle in an sich bekannter Weise miteinander kommunizieren. Die beiden Hörgeräte 4A, 4B sind zumindest weitgehend identisch aufgebaut. Auf Unterschiede wird explizit eingegangen.

Ein jeweiliges Hörgerät 4A, 4B weist jeweils einen oder mehrere Eingangswandler 6 auf, insbesondere Mikrofone, über die ein Eingangssignal, insbesondere ein akustisches Umgebungssignal, in ein elektrisches Eingangssignal E1, E2 umgewandelt wird. Dieses wird in einem Haupt-Signalpfad 8 und in einer Signalverarbeitungseinheit 10 zunächst in an sich bekannter Weise speziell auf Basis eines nutzerspezifischen Audiogramms und damit entsprechend einem nutzerspezifischen Hörschaden aufbereitet. Das aufbereitete elektrische Signal wird als elektrisches Ausgangssignal A1, A2 einem Ausgangswandler 12 zugeführt, welcher das elektrische Ausgangssignal in ein akustisches Ausgangssignal aA1, aA2 wandelt. Bei dem Ausgangswandler 12 handelt es sich insbesondere um einen Lautsprecher.

Die Signalverarbeitungseinheit 10 weist beispielsweise eingangsseitig eine Filterbank 14 auf, über die das elektrische Eingangssignal E1, E2 in unterschiedliche Frequenzbänder für die nachfolgende Signalbearbeitung zerlegt wird. Nachfolgend sind üblicherweise mehrere Signalverarbeitungseinheiten 16,18 wie beispielsweise ein Beamformer zur Erzeugung einer Direktionalität oder auch ein Rauschunterdrücker zur Reduzierung von störenden Umgebungsgeräuschen vorgesehen. Ein durch die Signalverarbeitungseinheiten 16,18 teilaufbereitetes elektrisches Eingangssignal E1, E2 wird anschließend einem Summierer 20 zugeführt. Nachfolgend zu dem Summierer 20 ist ein Signalverstärker 22 typischerweise mit integrierter Kompressionseinheit angeordnet. Dem Signalverstärker 22 ist schließlich beispielsweise eine synthetisierende Filterbank 24 nachgeordnet, die die einzelnen Frequenzbänder wieder zusammenführt und an dessen Ende das elektrische Ausgangssignal A1, A2 ausgegeben wird.

Neben dem Haupt-Signalpfad 8 weist ein jeweiliges Hörgerät 4A, 4B einen Neben-Signalpfad 26 auf. Dieser weist einen Signalgenerator 28 zur Erzeugung eines Beatsignals B1, B2 auf. Über den Signalgenerator 28 wird insbesondere zusätzlich zu dem elektrischen Eingangssignal E1, E2 ein weiteres Signal bereitgestellt. Bei diesem handelt es sich insbesondere um ein vom Eingangssignal unabhängiges Signal, speziell ein Sinussignal oder um ein insbesondere schmalbandiges Rauschsignal. Daneben besteht auch die Möglichkeit, als Basis für das Beatsignal B1, B2 ein Signalanteil des vom Eingangswandler 6 aufgenommen elektrischen Eingangssignals E1, E2, also Umgebungsgeräusche, heranzuziehen. Das Beatsignal B1, B2 setzt sich beispielsweise aus mehreren Signalanteilen zusammen, die auf Basis der oder durch die zuvor erwähnten Signale (Sinussignal, Rauschsignal, Umgebungsgeräusch) gebildet sind.

Zumindest in einem der beiden Signalgeneratoren 28, im Ausführungsbeispiel im ersten Hörgerät 4A, ist ein Frequenzverschieber 30 integriert, mit dessen Hilfe das zuvor generierte Signal (z.B. Sinussignal, Rauschsignal) um eine Beatfrequenz verschoben wird. Bei den beiden Beatsignalen B1, B2 handelt es sich daher um zwei identische Signale mit den gleichen Signalanteilen, die zueinander lediglich um die Beatfrequenz verschoben sind.

Weiterhin weist ein jeweiliges Hörgerät 4A, 4B jeweils eine Analyseeinheit 32 auf, die dazu eingerichtet ist, einen momentanen Signalpegel Lₘ des (akustischen) Eingangssignals zu ermitteln, insbesondere abzuschätzen. Hierzu wertet die Analyseeinheit 32 das vor dem Summierer 20 anliegende und ggf. teilweise aufbereitete elektrische Eingangssignal E1, E2 in geeigneter Weise aus.

In Abhängigkeit des dabei ermittelten momentanen, aktuell anliegenden Signalpegels Lₘ wird ein momentaner Verstärkungsfaktor bestimmt, mit dem das jeweilige Beatsignal B1, B2 verstärkt wird. Zur Verstärkung ist jeweils ein Verstärkerelement 34 angeordnet. Die Analyseeinheit 32 und das Verstärkerelement 34 sind Bauteile einer Einstellvorrichtung 36, über die ein jeweiliger Signalpegel Ls für das Beatsignal B1, B2 eingestellt wird.

Gemäß einer bevorzugten Ausgestaltung, wie sie in der Figur dargestellt ist, ist zumindest in einem der Hörgeräte 4A, 4B ein Synchronisierer 38 angeordnet, welcher mit den beiden Analyseeinheiten 32 verbunden ist und von diesen Information über den jeweiligen Signalpegel Lₘ erhält. Die Übermittlung dieser Information von dem einen Hörgerät 4B zu dem im anderen Hörgerät 4A angeordneten Synchronisierer 38 erfolgt insbesondere über die zuvor beschriebene Kommunikation-Schnittstelle des binauralen Hörsystems 2.

Der Synchronisierer 38 wertet die beiden momentanen Signalpegel Lₘ aus, vergleicht sie insbesondere und ermittelt auf Basis dieser Auswertung geeignete, synchronisierte Verstärkungsfaktoren für die beiden Beatsignale B1, B2. Bei stark unterschiedlichen momentanen Signalpegel Lₘ sorgt der Synchronisierer 38 insbesondere für eine Begrenzung der Unterschiede der Signalpegel L_{B} der Beatsignale B1, B2.

In einer alternativen, vereinfachten und hier nicht näher dargestellten Ausführungsvariante bestimmt jede Einstellvorrichtung 36 der beiden Hörgeräte 4A, 4B individuell einen jeweiligen momentanen Verstärkungsfaktor für das jeweilige Beatsignal B1, B2. Bei dieser Variante wird daher auf den Synchronisierer 38 und eine Synchronisation verzichtet.

Das jeweils verstärkte Beatsignal B1, B2 wird anschließend dem Summierer 20 zugeführt. Nachfolgend wird das resultierende, zusammengesetzte Signal, welches als Signalanteile das teilweise aufbereitete Eingangssignal E1, E2 sowie das Beatsignal B1, B2 aufweist, dem Signalverstärker 22 zugeführt, dort verstärkt und schließlich nach der synthetisierenden Filterbank 24 als das ausgehende elektrische Ausgangssignal A1, A2 bereitgestellt, welches anschließend durch den Ausgangswandler 12 in das akustische Ausgangssignal aA1, aA2 umgewandelt wird.

Das jeweilige akustische Ausgangssignal aA1, aA2 setzt sich daher aus einem normalen Audiosignalanteil und einem Beatsignalanteil zusammen. Der normale Audiosignalanteil, ist der Signalanteil, welcher sich ohne den jeweiligen Signalanteil der Beatsignale ergeben würde. Die Beatsignalanteile im akustischen Ausgangssignal aA1, aA2 sind zueinander um die Beatfrequenz frequenzverschoben und weisen ggf. unterschiedliche Amplituden auf, sind ansonst jedoch identisch.

Dem Nutzer werden daher an seinen beiden Ohren zusätzlich zu dem normalen Audiosignal zwei zueinander um die Beatfrequenz versetzte (akustische) Beatsignale B1, B2 bereitgestellt, so dass wie gewünscht die binauralen Beats vom Nutzer wahrgenommen werden.

Der momentane Signalpegel Lₘ wird vorzugsweise fortlaufend und vorzugsweise in Zeitintervallen von kleiner 300ms und beispielsweise alle 100ms erfasst und der Signalpegel L_{B} der Beatsignale B1, B2 wird kontinuierlich anhand der erfassten momentanen Signalpegel Lₘ nachgeführt. Und zwar vorzugsweise derart, dass sich im akustischen Ausgangssignal aA1, aA2 ein höherer Signalpegel L_{B} der Beatsignal-Anteile im Vergleich zu den Signalpegeln Lₘ des normalen Audiosignalanteils einstellt. Bevorzugt ist der Signalpegel L_{B} der Beatsignalanteile um 1dB bis 5dB und insbesondere um 3dB höher.

Vorliegend werden die elektrischen Eingangssignale über den ganzen Haupt-Signalpfad 8 einheitlich - unabhängig von ihrem Bearbeitungsstatus - mit dem Bezugszeichen E1, E2 versehen.

Vorliegend sind die vom jeweiligen Signalgenerator 28 bereitgestellten weiteren (Beat-) Signale über den gesamten Signalpfad und damit in unterschiedlichen Bearbeitungsstufen einheitlich mit dem jeweiligen Bezugszeichen B1, B2 bezeichnet.

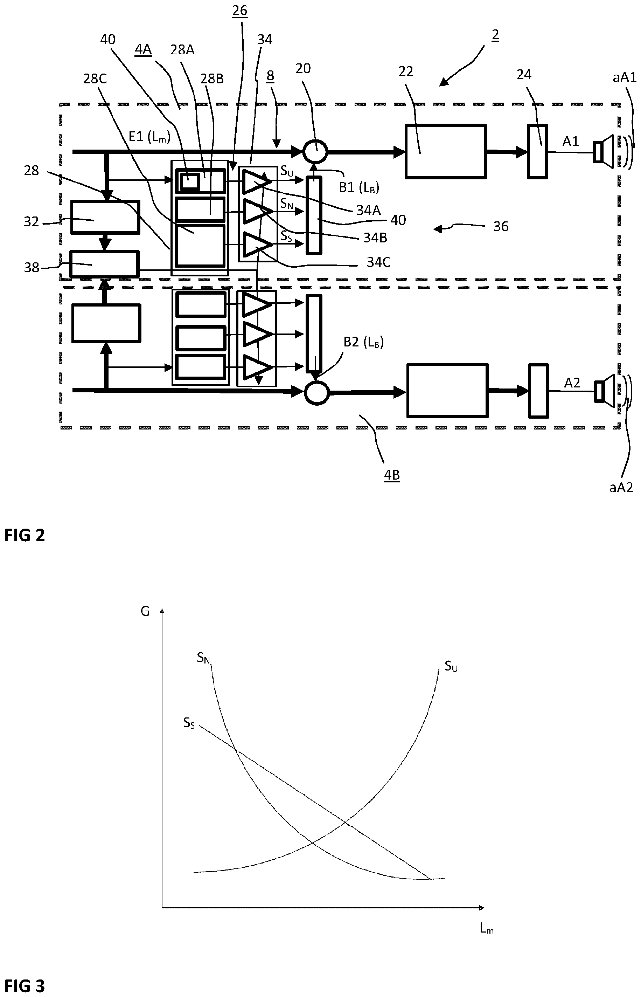

Figur 2 zeigt eine ausschnittsweise, vergrößerte Darstellung des Hörsystems 2 im Bereich des neben-Signalpfad 26.

Bei der Ausgestaltung gemäß der FIG 2 werden die Beatsignale B1, B2 aus unterschiedlichen Signalkomponenten zusammengesetzt. Eine Gewichtung G der verschiedenen Signalkomponenten erfolgt dabei in Abhängigkeit des Signalpegels Lₘ des akustischen Eingangssignals.

Der Signalgenerator 28 ist hierzu zunächst unterteilt in mehrere Generatorteile 28A, 28B und 28C. Ergänzend ist auch das Verstärkerelement 34 unterteilt in mehrere, individuell einstellbare Verstärker-Teilkomponenten 34A, 34B und 34C. Die verschiedenen Generatorteile 28A bis 28C sind dazu eingerichtet, unterschiedliche Signale als die Signalkomponenten zu generieren. Hierbei handelt es sich insbesondere um ein Umgebungssignal Su, ein Sinussignal Ss sowie ein schmalbandiges Rauschsignal S_{N}.

Das Generatorteil 28A dient zur Erzeugung des Umgebungssignals Su. Hierzu wird es eingangsseitig mit dem elektrischen Eingangssignal E1, E2 beaufschlagt. Es weist beispielsweise einen integrierten Bandfilter 40 auf, sodass aus dem Eingangssignal E1, E2 ein schmalbandiger Signalteil herausgeschnitten wird, welcher dann das Umgebungssignal Su bildet.

Die Generatorteile 28A-28C der beiden Hörgeräte 4A, 4B sind zueinander identisch ausgebildet mit dem Unterschied, dass in den Generatorteilen 28A bis 28C des ersten Hörgerätes 4A ergänzend noch ein hier nicht explizit dargestellter Frequenzverschieber 30 integriert ist, um die gewünschte Frequenzverschiebung um die Beatfrequenz zu erzeugen.

Die Generatorteile 28B, 28C erzeugen jeweils eigenständige Signale unabhängig von dem elektrischer Eingangssignal E1, E2.

Über die jeweilige Verstärker-Teilkomponente 34A bis 34C, die einen jeweiligen Generatorteil 28A bis 28C zugeordnet sind, erfolgt eine individuelle Verstärkung der verschiedenen Signalkomponente Su, S_{N}, Ss. Diese werden nachfolgend in einem Kombinierer 42, welcher insbesondere als Summierer ausgebildet ist, zusammengefügt und bilden dann das jeweilige Beatsignal B1, B2, welches dem Summierer 20 im Haupt-Signalpfad 8 zugeführt wird.

Die individuellen Verstärkungsfaktoren für die verschiedenen Signalkomponenten Su, S_{N}, Ss werden in Abhängigkeit des Signalpegels L_{M} des Eingangssignals eingestellt. Hierzu ist wie bereits zur FIG 1 beschrieben in jedem Hörgerät 4A, 4B eine Analyseeinheit 32 integriert, die zur Bestimmung und insbesondere Abschätzung des eingehenden Signalpegels L_{M} ausgebildet ist.

In FIG 2 ist analog zu FIG 1 eine Ausführungsvariante mit dem Synchronisierer 38 dargestellt. Dieser dient wiederum zur synchronisierten Ansteuerung des Verstärkerelements 34. Die Grundzüge sind dabei die gleichen wie zu FIG 1 beschrieben. Im Unterschied zu FIG 1 werden nunmehr jedoch die Verstärker-Teilkomponenten 34A bis 34C jeweils individuell angesteuert, sodass für die unterschiedlichen Signalkomponenten S_{U}, S_{N}, S_{S} unterschiedliche Verstärkungsfaktoren und damit Gewichtungen G eingestellt werden. Dies bedeutet, dass die verschiedenen Signalkomponente Su, S_{N}, Ss im dann zusammengesetzten Beatsignal B1, B2 unterschiedlich gewichtet sind. Die Signale B1, B2 weisen dann jeweils einen Signalpegel L_{B} auf. Die Verstärkungsfaktoren für die Signalkomponenten S_{U}, S_{N}, S_{S} werden dabei in geeigneter Weise derart gewählt, dass der gewünschte Wert für den Signalpegel Ls erreicht wird, wie er insbesondere im Zusammenhang mit der FIG 1 beschrieben wurde.

In einer alternativen Ausgestaltung ist auf den Synchronisierer 38 verzichtet und eine die Einstellung der Gewichtungen G erfolgt in den beiden Hörgeräten beispielsweise unabhängig voneinander und auf Basis jeweils des von der Analyseeinheit 32 des jeweiligen Hörgeräts 4A, 4B ermittelten Signalpegels Lₘ.

FIG 3 zeigt den qualitativen Verlauf der Gewichtung der unterschiedlichen Signalkomponenten S_{U}, S_{N}, S_{S} in Abhängigkeit des Signalpegels des Eingangssignals Lₘ. Auf der x-Achse ist dabei der Signalpegel L_{M} angegeben (nimmt von links nach rechts zu) und auf der Y-Achse ist die Gewichtung G und damit Verstärkung der verschiedenen Signalkomponenten S_{U}, S_{N}, S_{S} dargestellt.

Wie hieraus gut zu erkennen ist, dominiert bei einem niedrigen, leisen Signalpegel Lₘ das Rauschsignal S_{N}, dessen Gewichtung G mit zunehmendem Signalpegel Lₘ abnimmt und zwar vorzugsweise nach Art einer Hyperbel, die sich asymptotisch einem Minimalwert annähert, welcher vorzugsweise 0 ist oder alternativ ungleich 0.

Bei dem niedrigen, leisen Signalpegel Lₘ weist zudem das Sinussignal Ss noch eine - im Vergleich zu dem Umgebungssignal Su - hohen Gewichtungsfaktor auf, der jedoch geringer ist als der des Rauschsignals S_{N}. Der Gewichtungsfaktor des Sinussignals SS fällt mit zunehmendem Signalpegel Lₘ ab und zwar vorzugsweise linear.

Allgemein fällt das Rauschsignal S_{N} mit zunehmendem Signalpegel Lₘ stärker als das Sinussignal Ss ab, sodass sich deren beiden Kurven schneiden, sodass in einem mittleren Pegelbereich das Sinussignal Ss dominiert.

Im Unterschied zu diesen beiden Signalkomponente S_{N}, Ss steigt das Umgebungssignal Su mit zunehmendem Signalpegel Lₘ an. Der Anstieg verläuft beispielsweise exponentiell.

Im niedrigen, leisen Pegelbereich startet das Umgebungssignal Su bei einem konstanten Wert, insbesondere bei 0, sodass im leisen Pegelbereich sich das Beatsignal B1, B2 ausschließlich aus den beiden anderen Signalkomponenten S_{S}, S_{N} zusammensetzt.

Im mittleren Pegelbereich setzt sich demgegenüber das jeweilige Beatsignal B1, B2 aus allen drei Signalkomponenten Su, S_{N} Ss zusammen, wobei die Gewichtung innerhalb des mittleren Pegelbereich variiert. Als Beginn des mittleren Pegelbereich wird vorliegend der Schnittpunkt der Gewichtungskurven der Signalkomponenten Ss, S_{N} angesehen.

Im Mittleren Pegelbereich nimmt die Gewichtung des Umgebungssignals Su zunehmend zu, wobei sie zunächst beispielsweise die Gewichtung des Rauschsignals S_{N} und später auch Gewichtung des Sinussignals Ss überschreitet. Der Signalpegel Lₘ, ab dem der größte Gewichtungsanteil durch das Umgebungssignal Su gebildet wird, wird vorliegend als Ende des mittleren Regelbereichs definiert. Vorliegend ist es der Schnittpunkt zwischen der Gewichtungskurve des Sinussignals Ss und des Umgebungssignals Su.

Im sich an den mittleren Pegelbereich anschließenden hohen Pegelbereich nimmt die Gewichtung G des Umgebungssignals Su immer weiter zu, während die Gewichtung der beiden anderen Signale S_{N}, S_{S} weiter abnehmen und insbesondere auch bis auf 0 zurückgehen. In hohen Regelbereichen liegt daher vorwiegend (größer 75 % des Gesamtpegels) oder auch ausschließlich das Umgebungssignal Su an, sodass also das jeweilige Beatsignal B1, B2 vorwiegend oder ausschließlich durch das Umgebungssignal Su gebildet ist.

Die unterschiedliche Gewichtung der verschiedenen Signalkomponenten Su, S_{N}, Ss in Abhängigkeit des Signalpegels Lₘ beruht dabei auf der Erkenntnis, dass die unterschiedlichen Signalkomponenten Su, S_{N}, Ss in Abhängigkeit des Signalpegels Lₘ unterschiedlich wahrgenommen werden. So haben Untersuchungen gezeigt, dass bei niedrigen Signalpegel ein reiner Sinuston als unangenehm empfunden wird, wohingegen in einem mittleren Pegelbereich dieser im Vergleich zu einem Rauschsignal als angenehmer empfunden wird. Bei hohen Regelbereichen hat sich die Verwendung des Umgebungssignal Su als Signalkomponente als besonders vorteilhaft herausgestellt.

Bei den zuvor angeführten Einheiten wie Filterbank 14, 24, Signalverarbeitungseinheit 16, 18, Summierer 20, Signalverstärker 22, Signalgenerator 28 mit Generatorteilen 28A - 28C, Frequenzverschieber 30, Analyseeinheit 32, Verstärkerelement 34 mit Verstärker-Teilkomponenten 34A- 34C, Synchronisierer 38 sowie Bandfilter 40 handelt es sich jeweils um elektronische Bauteile oder Gruppen von elektronischen Schaltungsbauteilen, die im Betrieb die gewünschten beschriebenen Funktionen umsetzen. Diese Einheiten sind beispielsweise in einem oder in mehreren integrierten Schaltkreisen, Mikrochips und/oder Asics integriert.

### Bezugszeichenliste

- 2: Hörsystem
- 4A, B: Hörgerät
- 6: Eingangswandler
- 8: Haupt-Signalpfad
- 10: Signalverarbeitungseinheit
- 12: Ausgangswandler
- 14: Filterbank
- 16: Signalverarbeitungseinheit
- 18: Signalverarbeitungseinheit
- 20: Summierer
- 22: Signalverstärker
- 24: synthetisierende Filterbank
- 26: Neben-Signalpfad
- 28: Signalgenerator
- 28A- C: Generatorteile
- 30: Frequenzverschieber
- 32: Analyseeinheit
- 34: Verstärkerelement
- 34A-C: Verstärker-Teilkomponente
- 36: Einstellvorrichtung
- 38: Synchronisierer
- 40: Bandfilter

- E1, E2: elektrisches Eingangssignal
- A1, A2: elektrisches Ausgangssignal
- aA1, aA2: akustisches Ausgangssignal
- B1, B2: Beatsignal
- Lₘ: Signalpegel des Eingangssignals
- L_{B}: Signalpegel des Beatsignals
- Su: Umgebungssignalen
- Ss: Sinussignal
- S_{N}: Rauschsignal
- G: Gewichtung

## Patentansprüche

1. Hörsystem (2), insbesondere binaurales Hörsystem (2), welches zur Erzeugung von monauralen oder binauralen Beats ausgebildet ist, mit zumindest einem Hörgerät (4A, 4B), wobei
- das zumindest eine Hörgerät (4A, 4B) einen Haupt-Signalpfad (8) aufweist, mit einem Eingangswandler (6) zur Erzeugung eines elektrischen Eingangssignals (E1, E2) aus einem insbesondere akustischen Eingangssignal, mit einer Signalverarbeitungseinheit (10) zur Verarbeitung des elektrischen Eingangssignals (E1, E2) und zur Erzeugung eines elektrischen Ausgangssignals (A1, A2), sowie mit einem Ausgangswandler (12), welcher basierend auf dem elektrischen Ausgangssignal (A1, A2) ein akustisches Ausgangssignal (aA1, aA2) erzeugt,
- das zumindest eine Hörgerät (4A, 4B) weiterhin einen Neben-Signalpfad (26) aufweist, mit einem Signalgenerator (28) zur Erzeugung eines ersten Beatsignals (B1), wobei das erste Beatsignal (B1) zu einem weiteren Signal (B2) um eine Beatfrequenz frequenzverschoben ist, derart, dass im Betrieb beim Nutzer der Effekt des monauralen oder binauralen Beats erzeugt wird,
- das zumindest eine Hörgerät (4A, 4B) einen Summierer (20) aufweist, welcher zumindest das erste Beatsignal (B1) auf ein Signal des Haupt-Signalpfades (8) aufsummiert, wobei
- der Signalgenerator (28) mit dem ersten Signalpfad (8) verbunden ist und derart eingerichtet ist, dass das erste Beatsignal (B1) zumindest in bestimmten Betriebsmodi des Hörgerätes (4A, 4B) zumindest eine als Umgebungssignal (Su) bezeichnete Signalkomponente aufweist, die auf Basis des elektrischen Eingangssignal (E1) erzeugt ist.

2. Hörsystem (2) nach dem vorhergehenden Anspruch, welches als binaurales Hörsystem (2) und zur Erzeugung von binauralen Beats ausgebildet ist, mit dem zumindest einen, ersten Hörgerät (4A) und mit einem zweiten Hörgerät (4B), die für eine drahtlose, binaurale Kommunikation miteinander ausgebildet sind, wobei
- jedes der Hörgeräte (4A, 4B) einen Haupt-Signalpfad (8) aufweist, mit einem Eingangswandler (6) zur Erzeugung eines elektrischen Eingangssignals (E1, E2), mit einer Signalverarbeitungseinheit (10) zur Verarbeitung des Eingangssignals (E1, E2) und zur Erzeugung eines elektrischen Ausgangssignals (A1, A2), sowie mit einem Ausgangswandler (12), welcher basierend auf dem elektrischen Ausgangssignal (A1, A2) ein akustisches Ausgangssignal (aA1, aA2) erzeugt,
- jedes der Hörgeräte (4A, 4B) weiterhin einen Neben-Signalpfad (26) aufweist, mit jeweils einem Signalgenerator (28) zur Erzeugung des ersten Beatsignals (B1) im ersten Hörgerät (4A) und eines zweiten Beatsignals (B2) im zweiten Hörgerät (4B), wobei die beiden Beatsignale (B1, B2) zueinander um eine Beatfrequenz frequenzverschoben sind, derart, dass im Betrieb beim Nutzer der Effekt des binauralen Beats erzeugt wird,
- jedes der Hörgeräte (4A, 4B) einen Summierer (20) aufweist, welcher die Beatsignale (B1, B2) auf ein Signal des Haupt-Signalpfades (8) aufsummiert wobei
- jeder der Signalgeneratoren (38) mit jeweils dem ersten Signalpfad (8) verbunden ist und derart eingerichtet ist, dass das Beatsignal (B1, B2) zumindest in bestimmten Betriebsmodi des Hörgerätes (4A, 4B) zumindest eine als Umgebungssignal (Su) bezeichnete Signalkomponente aufweist, die auf Basis des elektrischen Eingangssignal (E1, E2) erzeugt ist.

3. Hörsystem (2) nach einem der vorhergehenden Ansprüche, bei dem das zumindest eine Hörgerät (4A, 4B) eine Analyseeinheit zur Analyse des Eingangssignals aufweist und das Beatsignal (B1, B2) sich aus ein oder mehreren Signalkomponenten (S_{U}, S_{N}, S_{S}) zusammensetzt, wobei der Signalgenerator (38) dazu eingerichtet ist, die Zusammensetzung des Beatsignals (B1, B2) auf Basis der Analyse des Eingangssignals vorzunehmen, wobei die Signalkomponenten (Su, S_{N}, Ss) bevorzugt ausgewählt sind aus einem Sinussignal (Ss), einem insbesondere schmalbandigen Rauschsignal (S_{N}) und dem aus dem elektrischen Eingangssignal abgeleiteten Umgebungssignal (Su).

4. Hörsystem (2) nach dem vorhergehenden Anspruch, bei dem die Analyseeinheit (32) zur Analyse eines Signalpegels (Lₘ) des Eingangssignals eingerichtet ist und die die Zusammensetzung des Beatsignals (B1, B2) auf Basis der Analyse des Eingangssignals vorgenommen wird

5. Hörsystem (2) nach dem vorhergehenden Anspruch, bei dem in Abhängigkeit des Signalpegels (Lₘ) eine unterschiedliche Gewichtung (G) der Signalkomponenten (S_{U}, S_{N}, S_{S}) vorgenommen wird, wobei die Gewichtung (G) des Umgebungssignals (Su) mit zunehmendem Signalpegel (Lₘ) vorzugsweise zunimmt.

6. Hörsystem (2) nach dem vorhergehenden Anspruch, bei dem bei geringen Signalpegeln (Lₘ) die Gewichtung (G) des Umgebungssignals (Su) geringer ist als die Gewichtung (G) jeder der anderen Signalkomponenten (S_{N}, S_{S}).

7. Hörsystem (2) nach einem der beiden vorhergehenden Ansprüche, bei dem in einem leisen Regelbereich des Signalpegels (Lₘ) das Rauschsignal (S_{N}), in einem mittleren Regelbereich das Sinussignal (Ss) und in einem hohen Regelbereich das Umgebungssignal (Su) am höchsten gewichtet wird.

8. Hörsystem (2) nach einem der Ansprüche 4 bis 7, bei dem die Analyse wiederkehrend durchgeführt wird und der Signalgenerator (28) dazu eingerichtet ist, die Zusammensetzung des Beatsignals (B1, B2) in Abhängigkeit der momentanen Analyse anzupassen.

9. Hörsystem (2) nach einem der Ansprüche 4 bis 8, bei dem neben dem Signalpegel (Lₘ) weitere Parameter als Bewertungskriterien für die Zusammensetzung des Beatsignals (B1, B2) und die Gewichtung (G) der Signalkomponenten (S_{U}, S_{N}, S_{S}) herangezogen werden.

10. Hörsystem (2) nach einem der vorhergehenden Ansprüche, bei dem das zumindest eine Hörgerät (4A, 4B) einen Bandfilter (40) aufweist zur Erzeugung eines bandgefilterten Umgebungssignals (Su), wobei das Umgebungssignal (Su) eine Bandbreite im Bereich von vorzugsweise 100 Hz bis 400 Hz aufweist und / oder wobei das Umgebungssignal (Su) bevorzugt in einem mittleren Bandbereich zwischen 200Hz und 1000Hz liegt..

11. Hörsystem (2) nach einem der Ansprüche 9 bis 10, bei dem Parameter des bandgefilterten Umgebungssignals (Su), wie Bandbreite, mittlere Frequenz oder untere bzw. obere Bandfrequenz in Abhängigkeit eines nutzerspezifischen Audiogramms eingestellt sind und / oder die Parameter des bandgefilterten Umgebungssignals (Su) im Zeitverlauf und insbesondere in Abhängigkeit des momentanen Eingangssignals variieren .

12. Hörsystem (2) nach einem der vorhergehenden Ansprüche, bei dem dem Signalgenerator (28) eine Einstellvorrichtung (36) zugeordnet ist, die ein einstellbares Verstärkerelement (34) aufweist, wobei die Einstellvorrichtung (36) eine Analyseeinheit (32) zur Bestimmung eines momentanen Signalpegels (Lₘ) des Eingangssignals aufweist, wobei die jeweilige Einstellvorrichtung (36) dazu eingerichtet ist, einen Signalpegel (L_{B}) des ersten Beatsignals (B1) in Abhängigkeit eines momentanen Signalpegels (Lₘ) des Eingangssignals (E1, E2) einzustellen.

13. Hörsystem (2) nach einem der vorhergehenden Ansprüche, **dadurch gekennzeichnet, dass** es für eine manuelle Einstellung des zumindest einen Beatsignals (B1, B1) durch den Nutzer ausgebildet ist

14. Hörsystem (2) nach einem der vorhergehenden Ansprüche, **dadurch gekennzeichnet, dass** es zur automatischen Identifizierung eines momentanen Status des Nutzers, insbesondere einen Schlafzustand des Nutzers ausgebildet ist und dass es zur Einstellung des zumindest einen Beatsignals (B1, B2) in Abhängigkeit des identifizierten momentanen Status ausgebildet ist.

15. Verfahren zur Erzeugung von monauralen oder binauralen Beats bei einem Hörsystem (2) mit zumindest einem Hörgerät (4A, 4B), wobei
- das zumindest eine Hörgerät (4A, 4B) einen Haupt-Signalpfad (8) aufweist, mit einem Eingangswandler (6) welcher ein elektrisches Eingangssignal (E1, E2) aus einem insbesondere akustischen Eingangssignal erzeugt, mit einer Signalverarbeitungseinheit (10), die das elektrische Eingangssignal (E1, E2) verarbeitet und ein elektrisches Ausgangssignal (A1, A2) erzeugt, sowie mit einem Ausgangswandler (12), welcher basierend auf dem elektrischen Ausgangssignal (A1, A2) ein akustisches Ausgangssignal (aA1, aA2) erzeugt,
- das zumindest eine Hörgerät (4A, 4B) weiterhin einen Neben-Signalpfad (26) aufweist, mit jeweils einem Signalgenerator (28), welcher ein erstes Beatsignal erzeugt, wobei das erste Beatsignal zu einem weiteren Signal um eine Beatfrequenz frequenzverschoben ist, derart, dass im Betrieb beim Nutzer der Effekt des monauralen oder binauralen Beats erzeugt wird,
- das zumindest eine Hörgerät (4A, 4B) einen Summierer (20) aufweist, welcher zumindest das erste Beatsignal auf ein Signal des ersten Signalpfades aufsummiert, wobei
- das Beatsignal (B1, B2) zumindest in bestimmten Betriebsmodi des Hörgerätes (4A, 4B) zumindest eine als Umgebungssignal bezeichnete Signalkomponente aufweist, die auf Basis des elektrischen Eingangssignal (E1, E2) erzeugt wird.
